# EUROPÄISCHE PATENTSCHRIFT

(11) **EP 3 632 886 B1**
(45) Veröffentlichungstag und Bekanntmachung des Hinweises auf die Patenterteilung: **22.06.2022**
(21) Anmeldenummer: 19200444.8
(22) Anmeldetag: 30.09.2019
(51) Int. Cl.: C07C 45/50, C07C 47/02, B01J 31/18, B01J 21/18, B01J 35/04, B01J 23/46, B01J 31/02, B01J 37/02

(54) **VERFAHREN ZUR HYDROFORMYLIERUNG VON KURZKETTIGEN OLEFINEN UNTER VERWENDUNG EINES HETEROGENISIERTEN KATALYSATORSYSTEMS OHNE IONISCHE FLÜSSIGKEIT**
METHOD FOR THE HYDROFORMYLATION OF SHORT CHAIN OLEFINS USING A HETEROGENISED CATALYST SYSTEM WITHOUT IONIC LIQUID
PROCÉDÉ D'HYDROFORMYLATION D'OLÉFINES À CHAÎNE COURTE À L'AIDE D'UN SYSTÈME CATALYTIQUE HÉTÉROGÉNÉISÉ SANS LIQUIDE IONIQUE

(30) Priorität: 05.10.2018 EP 18198787
(43) Veröffentlichungstag der Anmeldung: 08.04.2020
(73) Patentinhaber: Evonik Operations GmbH, 45128 Essen (DE)
(72) Erfinder: Hecht, Corinna, 45721 Haltern am See (DE); Franke, Robert, 45772 Marl (DE); Stenger, Frank, 63755 Alzenau (DE); Kreis, Peter, 44227 Dortmund (DE); Haßelberg, Jennifer, 44139 Dortmund (DE); Kristen, Marc Oliver, 45721 Haltern am See (DE)
(74) Vertreter: Evonik Patent Association

(56) Entgegenhaltungen:
- WO-A1-2015/028284
- DE-A1- 2 055 539
- ALBERTUS J. SANDEE ET AL: "ROTACAT: A Rotating Device Containing a Designed Catalyst for Highly Selective Hydroformylation", ADVANCED SYNTHESIS & CATALYSIS, Bd. 343, Nr. 2, 26. Februar 2001 (2001-02-26), Seiten 201-206, XP055576100, DE ISSN: 1615-4150, DOI: 10.1002/1615-4169(20010226)343:2<201::AID- ADSC201>3.0.CO;2-R

## Beschreibung

Die vorliegende Erfindung betrifft ein Verfahren zur Hydroformylierung von kurzkettigen Olefinen, insbesondere C2- bis C5-Olefinen, bei dem das Katalysatorssystem heterogenisiert auf einem Support aus einem porösen keramischen Material vorliegt, sowie Anlagen zur Durchführung dieses Verfahrens.

Die Hydroformylierung ist mit einer jährlichen globalen Produktionskapazität von mehreren Millionen Tonnen eine der bedeutendsten Reaktionen in der großtechnischen Chemie. Dabei werden Alkene (Olefine) mit einer Mischung aus Kohlenmonoxid und Wasserstoff (auch: Synthesegas oder Syngas) unter Verwendung eines Katalysators zu Aldehyden umgewandelt, die wichtige und wertvolle Zwischenprodukte bei der Herstellung von chemischen Massenprodukten wie Alkoholen, Estern oder Weichmachern sind.

Die Hydroformylierung wird im großtechnischen Maßstab ausschließlich homogen katalysiert durchgeführt. Die löslichen Übergangsmetallkatalysatorsysteme basieren üblicherweise auf Cobalt oder Rhodium, welches oft mit Phosphor-haltigen Liganden, bspw. Phosphinen oder Phosphiten, für die Hydroformylierung von eher kurzkettigen Olefinen eingesetzt wird.

Die Probleme bei den bekannten Verfahren sind vielfältig, die insbesondere damit verknüpft sind, dass sowohl Rhodium als auch Cobalt und deren Verbindungen vergleichsweise teuer sind. Es wird ein hoher energetischer und verfahrenstechnischer Aufwand betrieben, um Katalysatorverluste während des Hydroformylierungsprozesses weitestgehend zu vermeiden, beispielsweise durch teils sehr aufwändige Katalysatorrecyclingschritte. Zudem werden Produktaufreinigungsschritte aufwendiger, um sicherzustellen, dass möglichst keine Katalysatorrückstände im Produkt verbleiben.

Weitere Probleme bei den bekannten homogen katalysierten Verfahren sind die Stabilität der Liganden, die die Bedingungen der Hydroformylierung, wie Temperatur, Druck, pH-Wert, usw., überstehen müssen, und der Verbrauch von dem verwendeten Lösemittel während des Prozesses, der durch Nachdosieren ausgeglichen werden muss.

Um die vorgenannten Probleme bei der homogen katalysierten Hydroformylierung zu umgehen, sind Hydroformylierungsverfahren entwickelt worden, bei denen das Katalysatorsystem heterogenisiert wird, insbesondere durch Immobilisierung auf einem Trägermaterial (vgl. einleitende Diskussion in der WO 2015/028284 A1). Die Begriffe Heterogenisierung und Immobilisierung sind demnach so zu verstehen, dass der Katalysator durch Ausbildung eines dünnen Flüssigkeitsfilms mithilfe einer ionischen Flüssigkeit auf der Oberfläche und/oder in den Poren eines festen Trägermaterials immobilisiert wird und keine Reaktionslösung im klassischen Sinne vorliegt, in der der Katalysator homogen gelöst ist.

Eine Möglichkeit, den Katalysator in einer festen Phasen gehalten wird, ist in der DE 20 55 539 A1 offenbart. Die Hydroformylierung wird dabei insbesondere mit gasförmigen Olefinen wie Propylen durchgeführt, die in der Gasphase auf den festen Katalysator treffen.

Im Hinblick auf die Immobilisierung bzw. Heterogenisierung offenbart die bereits erwähnte WO 2015/028284 A1 sogenannte SILP-Systeme (SILP = Supported lonic Liquid Phase), bei der das Katalysatorsystem mit Rhodium, Iridium oder Cobalt als Zentralatom insbesondere auf einem porösen Siliciumdioxid-Träger unter Verwendung einer ionischen Flüssigkeit immobilisiert wird.

Es wird dabei angenommen, dass die ionische Flüssigkeit als Katalysatorträger bzw. als Lösemittel fungiert und dabei hilft das Katalysatorsystem auf dem Support zu immobilisieren. Das Problem mit ionischen Flüssigkeiten ist jedoch, dass diese vergleichsweise teuer und teilweise toxikologisch nicht unbedenklich sind.

Die Aufgabe der vorliegenden Erfindung war die Bereitstellung eines ein- oder mehrstufigen Hydroformylierungsverfahrens, bei dem in einem Hydroformylierungsschritt ein heterogenisiertes Katalysatorsystem verwendet wird, welches die vorgenannten Probleme nicht aufweist.

Gelöst wird diese Aufgabe gemäß Anspruch 1 dadurch, dass das ein- oder mehrstufige Hydroformylierungsverfahren mindestens einen Hydroformylierungsschritt umfasst, bei dem ein auf einem Support aus einem porösen keramischen Material heterogenisiertes Katalysatorsystem ohne ionische Flüssigkeit verwendet wird.

Ein Gegenstand der vorliegenden Erfindung ist somit ein Verfahren zur Hydroformylierung von C2-bis C5-Olefinen, wobei die Hydroformylierung einen oder mehrere Hydroformylierungsschritte umfasst, dadurch gekennzeichnet, dass
in mindestens einem Hydroformylierungsschritt ein Einsatzgemisch, welches die C2- bis C5-Olefine umfasst, mit Synthesegas in Gegenwart eines Katalysatorsystems, welches ein Metall aus der 8. oder 9. Gruppe des Periodensystems der Elemente, mindestens einen organischen Phosphor-enthaltenden Liganden und einen Stabilisator umfasst, in einer Reaktionszone einer Hydroformylierung unterworfen wird, wobei das Einsatzgemisch und das Synthesegas über einen Support aus einem porösen keramischen Material geleitet werden, auf dem das Katalysatorsystem heterogenisiert vorliegt,
das Katalysatorsystem keine ionische Flüssigkeit umfasst; und
der Support ein Monolith, das heißt ein Block aus einem keramischen Material ist, auf den ein Washcoat aus, bezogen auf das keramische Material des Supports, dem gleichen oder einem anderen keramischen Material aufgetragen wird,
eine organische Aminverbindung, die mindestens eine 2,2,6,6-Tetramethylpiperidineinheit nach Formel (I) enthält:

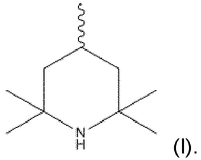

Als Einsatzgemisch können alle Gemische eingesetzt werden, die C2- bis C5-Olefine, insbesondere Ethen, Propen, 1-Buten, 2-Buten, 1-Penten oder 2-Penten, als Edukte umfassen. Die Menge an Olefinen in den Einsatzgemischen sollte verständlicherweise hoch genug sein, um eine Hydroformylierung wirtschaftlich betreiben zu können. Dazu gehören insbesondere technische Gemische der petrochemischen Industrie, wie beispielsweise Raffinatströme (Raffinat I, II oder III) oder Rohbutan. Rohbutan umfasst gemäß der vorliegenden Erfindung 5 bis 40 Gew.-% Butene, vorzugsweise 20 bis 40 Gew.-% Butene (die Butene setzen sich zusammen aus 1 bis 20 Gew.-% 1-Buten und 80 bis 99 Gew.-% 2-Buten) und 60 bis 95 Gew.-% Butane, vorzugsweise 60 bis 80 Gew.-% Butane.

Als Einsatzgemische können im Falle einer mindestens zweistufigen Hydroformylierung auch (gasförmige) Austräge aus einer anderen Hydroformylierung sein. Umfasst das erfindungsgemäße Verfahren mindestens zwei Hydroformylierungsschritte ist der Schritt, bei dem das auf einem Support aus einem porösen keramischen Material heterogenisierte Katalysatorsystem ohne ionische Flüssigkeit eingesetzt wird, mindestens der zweite Hydroformylierungsschritt. Die im ersten Hydroformylierungsschritt nicht umgesetzten Olefine können dann unter Verwendung des erfindungsgemäßen Katalysatorsystems noch umgesetzt werden. Zwischen den Hydroformylierungsschritten kann eine Stofftrennung angeordnet sein, mit der der gasförmige Austrag aus der ersten Hydroformylierung in mindestens eine an nicht umgesetzten Olefinen-reiche Phase und mindestens eine Produktaldehyd-reiche Phase getrennt wird. Die an nicht umgesetzten Olefinen-reiche Phase wird dann der zweiten Hydroformylierung unterworfen.

Das im erfindungsgemäßen Hydroformylierungsschritt eingesetzte Katalysatorsystem umfasst vorzugsweise ein Übergangsmetall aus der 8. oder 9. Gruppe des Periodensystems der Elemente, insbesondere Eisen, Ruthenium, Iridium, Cobalt oder Rhodium, besonders bevorzugt Cobalt und Rhodium, mindestens einen organischen Phosphor-enthaltenden Liganden und einen Stabilisator.

Der Stabilisator ist eine organische Aminverbindung, die mindestens eine 2,2,6,6-Tetramethylpiperidineinheit nach Formel (I) enthält:

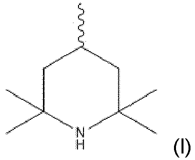

In einer bevorzugten Ausführungsform der vorliegenden Erfindung ist der Stabilisator ausgewählt aus der Gruppe, bestehend aus den Verbindungen der nachfolgenden Formeln (I.1), (I.2), (I.3), (I.4), (I.5), (I.6), (I.7) und (I.8). 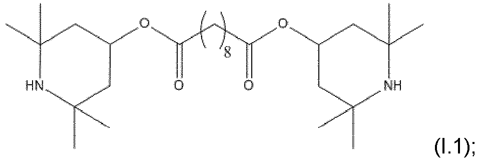 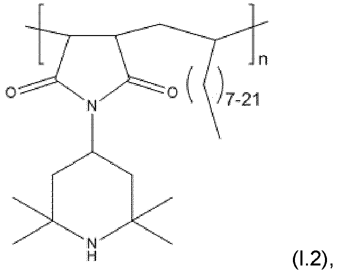 wobei n einer ganzen Zahl von 1 bis 20 entspricht; 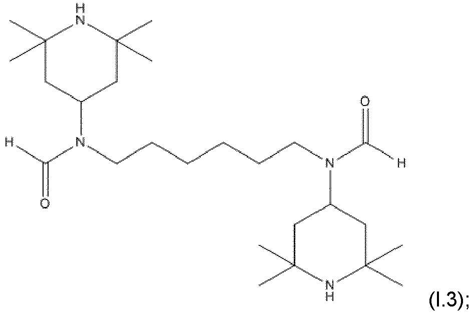 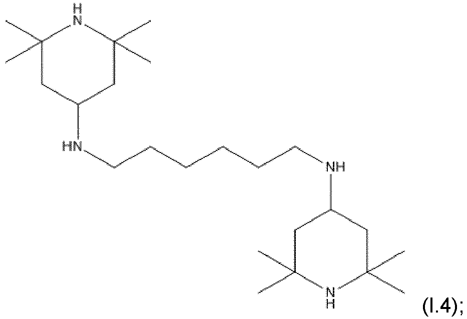 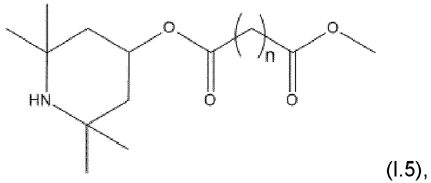 wobei n einer ganzen Zahl von 1 bis 12 entspricht; 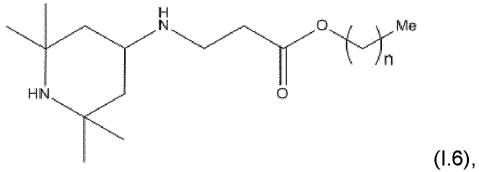 wobei n einer ganzen Zahl von 1 bis 17 entspricht; 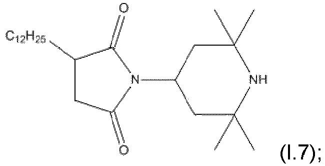 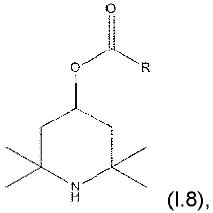 wobei R einer C6- bis C20-Alkylgruppe entspricht.

Für alle filmbildenden Komponenten, d. h. in diesem Fall der Stabilisator, sollte die Gaslöslichkeit für die Reaktanden besser sein als die Gaslöslichkeit der Produkte. Bereits dadurch kann eine partielle Stofftrennung zwischen eingesetzten Eduktolefinen und gebildeten Produktaldehyden erreicht werden. Grundsätzlich wären dafür auch andere filmbildende Substanzen denkbar, allerdings ist darauf zu achten, dass es nicht zu einer erhöhten Hochsiederbildung kommt und/oder die Nachführung der Eduktolefine eingeschränkt wird.

Der organische Phosphor-enthaltende Ligand für das Katalysatorsystem weist vorzugsweise die allgemeine Formel (VI)

R'-A-Rʺ-A-R‴ (VI)

wobei R′, Rʺ und R‴ jeweils organische Reste sind, mit der Maßgabe das R′ und R‴ nicht identisch sind, und beide A jeweils eine brückende -O-P(-O)₂-Gruppe sind, wobei zwei der drei Sauerstoffatome -O-jeweils an Rest R' und den Rest R‴ gebunden sind. Die organischen Reste R', R" und R‴ enthalten vorzugsweise keine endständige Trialkoxysilangruppe.

In einer bevorzugten Ausführungsform sind R', R" und R‴ in der Verbindung der Formel (VI), vorzugsweise ausgewählt aus substituierten oder unsubstituierten 1,1'-Biphenyl-, 1,1'-Binaphthyl-und ortho-Phenylgruppen, insbesondere aus substituierten oder unsubstituierten 1,1'-Biphenylgruppen, mit der Maßgabe das R′ und R‴ nicht identisch sind. Besonders bevorzugt weisen die substituierten 1,1'-Biphenylgruppen in 3,3'- und/oder 5,5'-Stellung des 1,1'-Biphenylgrundkörpers eine Alkylgruppe und/oder eine Alkoxygruppe auf, insbesondere eine C1-C4-Alkylgruppe, besonders bevorzugt eine tert.-Butyl- und/oder Methylgruppe und/oder bevorzugt einen C1-C5-Alkoxgruppe, besonders bevorzugt eine Methoxygruppe.

Das vorgenannte Katalysatorsystem liegt erfindungsgemäß heterogenisiert auf einem Support aus einem porösen keramischen Material vor. Im Sinne der vorliegenden Erfindung ist der Ausdruck "heterogenisiert auf einem Support" so zu verstehen, dass das Katalysatorsystem durch Ausbildung eines dünnen, festen oder flüssigen Films mithilfe des Stabilisators auf der inneren und/oder äußeren Oberfläche eines festen Trägermaterials immobilisiert wird. Der Film kann auch bei Raumtemperatur fest und bei Reaktionsbedingungen flüssig sein.

Die innere Oberfläche des festen Trägermaterials umfasst insbesondere die innere Oberfläche der Poren und/oder Kanäle. Immobilisierung umfasst begrifflich sowohl den Fall, dass das Katalysatorsystem und/oder die katalytisch aktive Spezies gelöst in dem festen oder flüssigen Film vorliegen, als auch die Fälle, dass der Stabilisator als Haftvermittler wirkt oder dass das Katalysatorsystem auf der Oberfläche adsorbiert wird, nicht jedoch chemisch bzw. kovalent gebunden auf der Oberfläche vorliegt.

Es liegt erfindungsgemäß also keine Reaktionslösung im klassischen Sinne vor, in der der Katalysator homogen gelöst ist, sondern das Katalysatorsystem befindet sich dispers verteilt auf der Oberfläche und/oder in den Poren des Supports.

Das poröse keramische Material ist vorzugsweise ausgewählt aus der Gruppe, bestehend aus einer Silikatkeramik, einer oxidischen Keramik, einer nitridischen Keramik, einer carbidischen Keramik, einer silicidischen Keramik und Mischungen davon.

Die Silikatkeramik ist vorzugsweise ausgewählt aus Alumosilikat, Magnesiumsilikat, und Mischungen davon, wie z. B. Bentonit. Die oxidische Keramik ist vorzugsweise ausgewählt aus γ-Aluminiumoxid, α-Aluminiumoxid, Titandioxid, Beryliumoxid, Zirkoniumoxid, Aluminiumtitanat, Bariumtitanat, Zinkoxid, Eisenoxide (Ferrite) und Mischungen davon. Die nitridische Keramik ist vorzugsweise ausgewählt aus Siliciumnitrid, Bornitrid, Aluminiumnitrid und Mischungen davon. Die carbidische Keramik ist vorzugsweise ausgewählt aus Siliciumcarbid, Borcarbid, Wolframcarbid oder Mischungen davon. Denkbar sind auch Mischungen aus carbidischen und nitridischen Keramik, die sogenannten Carbonitride. Die silicidische Keramik ist vorzugsweise Molybdändisilicid. Der Support gemäß der vorliegenden Erfindung, auf den das Katalysatorsystem aufgebracht wird, besteht vorzugsweise aus einer carbidischen Keramik.

Der Support ist ein Monolith, das heißt der Support aus dem porösen keramischen Material besteht aus einem Block (ein dreidimensionales Objekt) aus einem keramischen Material. Der Block kann sowohl einstückig ausgebildet sein als auch aus mehreren, also mindestens zwei Einzelteilen bestehen, die zu dem Block zusammengefügt werden können und/oder miteinander fest oder lösbar verbunden sind. Der Support ist aber insbesondere kein Granulat, welches als Katalysatorschüttung in Festbettreaktoren eingesetzt werden kann.

Der Support aus dem porösen keramischen Material ist vorzugsweise ein sich dreidimensional erstreckendes Bauteil, welches in seinem Querschnitt grundsätzlich beliebige geometrische Formen, beispielsweise rund, eckig, quadratisch o. ä., aufweisen kann. Das sich dreidimensional erstreckende Bauteil, welches als Support eingesetzt werden kann, weist in einer bevorzugten Ausführungsform eine Längsrichtung (Richtung der längsten Ausdehnung) in Hauptdurchströmungsrichtung (Richtung in die das Einsatzgemisch und das Synthesegas von Einlass zum Auslass des Reaktors strömen) auf.

Der so geformte Support aus dem porösen keramischen Material weist zumindest einen durchgängigen Kanal in Hauptdurchströmungsrichtung auf. Der oder die Kanäle können jedoch auch so ausgestaltet sein, dass sie nicht komplett durchgängig sind, sondern zu dem Ende, welches dem Einlass des Reaktors gegenüberliegt, einen Abschluss aufweisen bzw. der Kanal zu diesem Ende hin geschlossen ist. Der Support kann auch mindestens zwei oder mehr Kanäle aufweisen. Der Durchmesser der Kanäle kann im Bereich von 0,25 bis 50 mm, vorzugsweise im Bereich von 1 bis 30 mm, weiterhin bevorzugt im Bereich von 1,5 bis 20 mm und besonders bevorzugt im Bereich von 2 bis16 mm liegen. Sind mehrere Kanäle vorhanden, können die Durchmesser der Kanäle gleich oder verschieden voneinander sein. Der Durchmesser der Kanäle ist im Vergleich zu dem oder zu einem der Durchmesser des gesamten Supports insbesondere so zu wählen, dass die mechanische Stabilität nicht beeinträchtigt wird.

Darüber hinaus ist der Support aus dem keramischen Material porös, weist also Poren auf. Das erfindungsgemäße Katalysatorsystem befindet sich insbesondere auch in dem festen oder flüssigen Film in diesen Poren. Der Porendurchmesser liegt vorzugsweise im Bereich von 0,9 nm bis 30 µm, vorzugsweise im Bereich von 10 nm bis 25 µm und besonders bevorzugt im Bereich von 70 nm bis 20 µm. Der Porendurchmesser kann mittels Stickstoffadsorption oder Quecksilber-Porosimetrie gemäß DIN 66133 (Stand: 1993-06) bestimmt werden.

In einer bevorzugten Ausführungsform weist der Support zumindest teilweise durchgängige Poren auf, die sich von der Oberfläche zu den Kanälen hin und/oder von einem Kanal zu dem oder zu den nächstliegenden Kanälen erstrecken. Möglich ist auch das mehrere Poren miteinander verbunden sind und so insgesamt eine einzige durchgängige Pore bilden.

Die Herstellung des Supports aus einem porösen keramischen Material, auf dem das Katalysatorsystem heterogenisiert vorliegt, erfolgt wie nachfolgend beschrieben: Auf den Support aus dem keramischen Material wird zusätzlich ein sogenannter Washcoat aufgetragen, welcher bezogen auf das keramische Material des Supports aus dem gleichen oder einem unterschiedlichen Keramikmaterial, insbesondere ein aus den vorgenannten Keramikmaterialen ausgewähltes Keramikmaterial, vorzugsweise Siliciumoxid besteht. Der Washcoat selber kann porös oder unporös sein, vorzugsweise ist der Waschcoat unporös. Die Partikelgröße des Washcoats beträgt vorzugsweise 5 nm bis 3 µm, vorzugsweise 7 nm bis 700nm. Der Washcoat wird verwendet, um die gewünschte Porengröße einzubringen oder zu generieren und/oder, um die Oberfläche des Supports zu erhöhen. Der Auftrag des Washcoats kann insbesondere mittels Eintauchen (Dipcoating) in eine Washcoat-Lösung, die das Keramikmaterial des Washcoats, ggf. auch als Precursor, enthält, erfolgen. Die Menge das auf dem Support befindlichen Waschcoats beträgt ≤ 20 Gew.-%, vorzugsweise ≤ 15 Gew.-%, besonders bevorzugt ≤ 10 Gew.-% bezogen auf die Gesamtmenge des Supports.

Auf den so erzeugten keramischen Support mit dem aufgebrachten Washcoat wird das Katalysatorsystem aufgebracht. Dazu wird zunächst eine Katalysatorlösung durch Mischen, insbesondere bei Raumtemperatur und Umgebungsdruck, hergestellt, die mindestens einen organischen Phosphor-haltigen Ligand, mindestens einen Metall-Precursor, beispielsweise Chloride, Oxide, Carboxylate des jeweiligen Metalls, mindestens einen Stabilisator und mindestens ein Lösemittel umfasst. Die Katalysatorlösung wird erfindungsgemäß explizit ohne ionische Flüssigkeit angesetzt werden. Die Herstellung der Katalysatorlösung sollte insbesondere in einer inerten Umgebung, bspw. einer Glovebox erfolgen. Inerte Umgebung heißt in diesem Fall eine möglichst Wasser- und Sauerstoff-freie Atmosphäre.

Das Lösemittel kann aus allen Lösemittelklassen gewählt werden (protisch, aprotisch, polar oder unpolar) Voraussetzung für das Lösemittel ist die Löslichkeit von Katalysatorsystem (Ligand, Metall Precursor und Stabilisator) und bevorzugt auch der bei der Hydroformylierung entstehenden Hochsieder. Die Löslichkeit kann innerhalb des Immobilisierungsschrittes durch Aufheizen erhöht werden.

Das Lösemittel ist vorzugsweise aprotisch, polar, wie z. B. Acetonitril und Ethylacetat oder aber auch aprotisch, unpolar wie z. B. THF und Dietehylether. Auch Chlorkohlenwasserstoffe wie z. B. Dichlormethan, können als Lösemittel verwendet werden.

Die so hergestellte Katalysatorlösung wird dann mit dem Support (optional inkl. Washcoat) in Kontakt gebracht, beispielsweise durch Eintauchen (Dip-Coating) oder mittels Befüllen in einem Druckgefäß, beispielsweise direkt im Reaktor (in-situ-Imprägnierung). Sofern das Aufbringen der Katalysatorlösung außerhalb des Reaktors erfolgt, muss der Support nach Abtrennen des Lösemittels natürlich wieder in den Reaktor eingebaut werden. Bevorzugt wird die Katalysatorlösung direkt im Reaktor auf den Support mit dem Washcoat aufgebracht, weil dadurch möglicherweise zeitaufwändige Ein- und Ausbauschritte sowie eine mögliche Kontamination des Katalysators vermieden werden können.

Bei der in-situ-Imprägnierung wird der Reaktor vor dem Befüllen mit einem Inertgas, beispielsweise Edelgase, Alkane oder Stickstoff, gespült. Das Spülen kann bei 1 bis 25 bar durchgeführt werden, vorzugsweise unter leichtem Überdruck von 20 bis 90 mbar, besonders bevorzugt 30 bis 60 mbar über Normaldruck. Der Reaktor kann vor dem Spülen mit Inertgas abgekühlt werden, um zu verhindern, dass das Lösemittel der einzufüllenden Katalysatorlösung sofort verdampft. Weist das Lösemittel jedoch eine Siedetemperatur auf, die größer ist als die Temperatur des Reaktors kann das Abkühlen des Reaktors entfallen.

Nach dem Spülen mit Inertgas kann der vorhandene Druck, beispielsweise über die Druckregelung, abgelassen werden, vorzugweise bis der Reaktor drucklos ist, also Umgebungsdruck (ca. 1 bar) aufweist. Andererseits kann in dem Reaktor, auch ein Vakuum erzeugt werden, beispielsweise mit einer Vakuumpumpe. In einer Ausgestaltung der vorliegenden Erfindung kann der Reaktor, nach dem Ablassen des Drucks oder nach dem Ziehen des Vakuums erneut mit einem Inertgas wie oben beschrieben gespült werden. Dieser Vorgang von Druck ablassen / Vakuum ziehen und erneutem Spülen kann beliebig oft wiederholt werden.

Die Katalysatorlösung wird für das Befüllen des Reaktors in einem Druckgefäß vorgelegt und vorzugsweise mit einem Inertgasüberdruck von 1 bis 25 bar, besonders bevorzugt einem leichten Inertgasüberdruck von 20 bis 90 mbar, vorzugsweise 30 bis 60 mbar über Reaktordruck beaufschlagt. Das Inertgas kann ein Edelgas, ein Alkan, beispielsweise Butan, oder Stickstoff sein. Die Katalysatorlösung wird dann mit dem genannten Überdruck, mit dem das Druckgefäß beaufschlagt ist, insbesondere druckgetrieben in den Reaktor eingefüllt. Der Druck des Druckgefäßes sollte beim Befüllen höher sein als im Reaktor. Dabei können Temperaturen im Bereich von 20 bis 150 °C und ein Druck von 1 bis 25 bar vorliegen.

Eine andere Möglichkeit des Befüllens ist, dass der Reaktor nach dem Spülen mit Inertgas im Vakuum gehalten wird und die Katalysatorlösung durch den Unterdruck in den Reaktor gezogen wird. Dabei sollte für die Herstellung der Katalysatorlösung ein Lösemittel verwendet werden, welches bei dem herrschenden Vakuum bzw. Unterdruck und den herrschenden Temperaturen siedet.

Das Befüllen des Reaktors mit der Katalysatorlösung kann über die normalen Ein- bzw. Ausgänge erfolgen. Flüssigkeitsverteiler oder Düsen innerhalb des Reaktors können für eine gleichmäßige Verteilung der Katalysatorflüssigkeit sorgen, ebenso wie optional vorliegende Druckverlusteinbauten oder Regelungen für die Dosiergeschwindigkeit. Nach dem Aufbringen des Katalysatorsystems wird das Lösemittel abgetrennt. Dabei wird zunächst die restliche Katalysatorlösung über den Auslass des Reaktors abgelassen. Danach werden im Reaktor verbliebene Lösemittelreste durch Einstellen des Drucks oder Erhöhung der Temperatur verdampft. Die Einstellung des Drucks kann in einer anderen Ausführungsform auch unter gleichzeitiger Erhöhung der Temperatur durchgeführt werden. Die Temperatur kann in Abhängigkeit vom Lösemittel 20 bis 150 °C betragen. Der Druck kann in Abhängigkeit vom Lösemittel auf ein Hochvakuum (10⁻³ bis 10⁻⁷ mbar) eingestellt werden, je nach Lösemittel und Temperatur sind aber auch Überdrücke von einigen mbar bis zu mehreren bar denkbar.

Der Stabilisator verbleibt mit dem Katalysator aus dem Metall, insbesondere Cobalt oder Rhodium, und dem organischen Phosphor-haltigen Liganden heterogenisiert auf dem Support.

Das Aufbringen des Katalysatorsystems auf den Support kann sowohl direkt im Reaktor (in situ) oder außerhalb des Reaktors erfolgen. Das Aufbringen des Katalysatorsystems wird in einer bevorzugten Ausführungsform der vorliegenden Erfindung direkt im Reaktor, also in situ, aufgebracht. Nach dem Abtrennen des Lösemittels kann der Reaktor sofort eingesetzt werden und mit dem Einsatzgemisch beschickt werden. Das hat den Vorteil, dass keine zeitaufwändigen Ein- und Ausbauschritte notwendig sind, die einen längeren Ausfall des Reaktors zur Folge hätten. Zudem ist die Größe des Supports dann nicht mehr dadurch limitiert, dass geeignete Räumlichkeiten mit inerten Umgebungen einer bestimmten Größe vorliegen. Die Größe des Supports kann in Abhängigkeit vom Reaktordesign frei gewählt werden. Ein weiteres Problem ist, dass der Support immer unter Luftabschluss transportiert werden muss, was teilweise schwer zu realisieren ist.

Nach erfolgtem Aufbringen des Katalysatorsystems auf den Support und erfolgter Abtrennung des Lösemittels kann die Anlage, insbesondere der Reaktor durch eine zwei- oder mehrstufige Anfahrprozedur hochgefahren werden, also in den Betrieb überführt werden.

Das Ziel der Anfahrprozedur ist eine schonende Aktivierung des Katalysatorsystems und ein Abfedern der maximalen Startaktivität des Katalysators zur Verlängerung der Lebenszeit des Katalysatorsystems. Außerdem soll durch die Anfahrprozedur die Ausbildung einer Flüssigphase verhindert werden, da dies zu einer Desaktivierung, Blockierung und oder zum Auswaschen des Katalysatorsystems führen kann. Besonders beim Anfahren eines frisch hergestellten Katalysatorsystems (auf dem Support) mit konzentriertem Edukt kann nämlich ein Reaktionsumsatzmaximum erreicht werden, welches auch mit einer maximalen Nebenproduktbildung (Hochsieder) verbunden ist. Übersteigt der Anteil der hochsiedenen Nebenprodukte in Abhängigkeit von den Betriebsbedingungen (Druck und Temperatur) einen gewissen Wert, kann dies aufgrund der von der vorliegenden Mischung abhängigen Dampfdrücke der Einzelkomponenten in der Ausbildung einer Flüssigphase resultieren, die das Katalysatorsystem schädigen, blockieren oder auswaschen kann.

Die Aktivierung des Katalysatorsystems wird erfindungsgemäß vorzugsweise mit einer zeitlich verlängerten Umsatzsteigerung realisiert. So lässt sich für jede Kombination aus Druck, Temperatur und Zusammensetzung des Einsatzgemisches ein maximal zulässiger Umsatz für die Bildung von Nebenprodukten berechnen, der nicht überschritten werden darf, um die vorgenannten Probleme nicht entstehen zu lassen. Der Umsatz für die Bildung von Nebenprodukten kann auch in Abhängigkeit des Umsatzes für die Bildung der Produktaldehyde (= in Abhängigkeit der Aldehydkonzentration) ermittelt werden, d. h. die Anfahrprozedur richtet sich nach dem maximalen Umsatz der Einsatzolefine.

Bei bekannten Langzeit-Betriebsbedingungen des Reaktors, die einen zuverlässigen Umsatzgrad der Einsatzolefine von 20 bis 95%, vorzugsweise von 80% - 95% ermöglichen, kann die Anfahrprozedur so realisiert werden, dass die Zusammensetzung des Einsatzgemisches, welches in den Reaktor gefahren wird, stufenweise geändert wird, ohne dass der maximale Umsatz der Einsatzolefine überschritten wird.

Dabei kann die Zusammensetzung des Einsatzgemisches, die bei Langzeit-Betriebsbedingungen für einen zuverlässigen Umsatz der Olefine sorgt, so variiert werden, dass bei konstantem Volumenstrom der Olefin- und/oder der Synthesegasanteil in mindestens zwei, vorzugsweise mehr als drei Stufen, insbesondere vier oder mehr Stufen, angehoben wird, ohne dass der maximale Umsatz der Einsatzolefine überschritten wird. Dem technischen Einsatzgemisch und Synthesegasgemisch können dazu in der oder den ersten Stufe(n) Inertgase wie z.B. N₂, Argon, Helium oder ähnliches zugeführt wird.

Mit fortschreitender Betriebszeit kann die Aktivität des Katalysators abnehmen, beispielsweise durch die Anreicherung von Hochsiedern und/oder der Belegung oder Desaktivierung von aktiven Zentren. Die Hochsieder können zu einer verstärkten Kondensation in den Poren führen, sodass die Poren nicht mehr oder langsamer für die Eduktolefine zugänglich sind. Andererseits können einige Nebenprodukte zu einem Zerfall des Katalysatorsystems führen, wodurch die Aktivität des Katalysators ebenfalls abnimmt. Eine Abnahme der Katalysatoraktivität kann beispielsweise anhand des Absinkens von Umsätzen oder Selektivitäten, insbesondere durch eine entsprechende Analytik mittels Ramanspektroskopie, Gaschromatographie oder Massendurchflussmesser (MDM) ermittelt werden. Möglich wäre auch ein modellbasiertes Überwachen der Katalysatoraktivität. Dies würde eine von den Betriebsbedingungen unabhängige Methode zur Überwachung der Katalysatoraktivität darstellen, aber auch um den Verlauf zu extrapolieren und damit eine Revisions-/Regenerationsplanung zu unterstützen.

In dem Fall einer unzureichenden Katalysatoraktivität kann das Katalysatorsystem, welches heterogenisiert auf dem porösen keramischen Support vorliegt, ausgetauscht werden. Dazu kann der Reaktor bzw. der Support in dem Reaktor einmalig oder mehrmalig mit einem Lösemittel gespült werden. Durch das Spülen kann das Katalysatorsystem demobilisiert und entfernt werden. Das Lösemittel kann eines der für die Herstellung der Katalysatorlösung genannten Lösemittel sein. Die Temperatur beim Spülen mit Lösemittel kann 20 bis 150 °C betragen. Der Druck kann beim Spülen mit Lösemittel zudem 1 bis 25 bar betragen.

Nach dem Spülen wird der Support ein- oder mehrfach neu imprägniert, insbesondere mit der vorher beschriebenen in-situ-Imprägnierung des Supports. Die in-situ-Imprägnierung wird damit erneuert und das heterogenisierte Katalysatorsystem frisch aufgebracht. Die erneute in-situ-Imprägnierung kann unter genau den gleichen Bedingungen durchgeführt werden, wie sie für die erste in-situ-Imprägnierung oben beschrieben worden sind

Aufgrund der Tatsache, dass das Katalysatorsystem durch Spülen und erneutes Aufbringen vollständig ausgetauscht wird, können diese Schritte ständig wiederholt werden, sobald die Aktivität des Katalysators erneut absinkt. Ein weiterer Vorteil ist, dass sowohl Hochsieder und Produktaldehyde als auch Zerfallsprodukte des Katalysatorsystems ausgeschleust werden können. Es sollte jedoch darauf geachtet werden, dass die Eigenschaften des Supports durch Demobilisierung und erneute in-situ-Imprägnierung nicht beeinträchtigt werden. Andernfalls wäre auch ein Austausch des porösen keramischen Supports durchzuführen.

Eine weitere Option ist, dass der gesamte poröse keramische Support, auf dem das Katalysatorsystem heterogenisiert vorliegt, ausgetauscht wird. Das Katalysatorsystem, welches auf dem (aus dem Reaktor entfernten) Support heterogenisierten vorliegt, kann dann außerhalb des Reaktors wie oben beschrieben ausgetauscht und bis zum nächsten Einbau und Einsatz im Reaktor gelagert werden. Wie erwähnt ist beim Auftrag des Katalysatorsystems eine inerte Umgebung erforderlich, weshalb die Handhabung und Lagerung bei der genannten Verfahrensweise von Aus- und Einbau des Supports unter entsprechenden Bedingungen erfolgen sollte.

Grundsätzlich ist eine Verfahrensführung bevorzugt bei der in der Reaktionszone mehrere Reaktoren parallel vorliegen und diese alternierend verwendet werden. Dabei wird mindestens ein Reaktor (a) für die erfindungsgemäße Hydroformylierung eingesetzt, also einer, der in Betrieb ist, und mindestens ein Reaktor (b), der in Wartehaltung ist. Das ist so zu verstehen, dass sobald bei dem sich in Betrieb befindende Reaktor (a) eine nicht mehr ausreichende Katalysatoraktivität festgestellt wird, der Strom des Einsatzgemisches von diesem Reaktor (a) auf den nächsten Reaktor (b) in Wartehaltung, der damit in Betrieb genommen wird, umgeschaltet wird. Reaktor (a) wird dann in Regenerationsmodus überführt, wo das Katalysatorsystem wie oben beschrieben regeneriert oder demobilisiert und der Support neu imprägniert wird, und dann in die Warteposition überführt bis ein erneuter Wechsel mit Reaktor (b) erfolgt. Dies Prinzip lässt sich auch auf 3 oder mehr Reaktoren anwenden, wobei mindestens ein Reaktor in Betrieb ist, einer oder mehre Reaktoren gleichzeitig in Wartehaltung sind und einer oder mehrere Reaktoren gleichzeitig im Regenerationsmodus sind.

Der zweite Hydroformylierungsschritt wird vorzugsweise bei den folgenden Bedingungen durchgeführt: Die Temperatur beim zweiten Hydroformylierungsschritt sollte im Bereich von 65 bis 200 °C, vorzugsweise 75 bis 175 °C und besonders bevorzugt 85 bis 150 °C liegen. Der Druck sollte 35 bar, vorzugsweise 30 bar, besonders bevorzugt 25 bar während des zweiten Hydroformylierungsschritts nicht überschreiten. Das molare Verhältnis zwischen Synthesegas und dem zweiten Einsatzgemisch sollte zwischen 6:1 und 1:1 liegen. Optional kann das zweite Einsatzgemisch mit Inertgas verdünnt werden, beispielsweise den in technischen Kohlenwasserstoffströmen befindlichen Alkanen.

Aus der Reaktionszone, in der die erfindungsgemäße Hydroformylierung durchgeführt wird, wird vorzugsweise kontinuierlich ein gasförmiger Austrag entnommen, der mindestens einen Teil der entstandenen Produktaldehyde und mindestens einen Teil der nicht umgesetzten Olefine enthält. Der gasförmige Austrag kann einem oder mehreren Stofftrennungsschritt(en) unterworfen werden, bei der der gasförmige Austrag in mindestens eine an nicht umgesetzten Olefinen-reiche Phase und mindestens eine Produktaldehyd-reiche Phase getrennt wird.

Die Stofftrennung kann mit bekannten Stofftrennungsverfahren, wie Kondensation, Destillation, Zentrifugation, Nanofiltration oder Kombination von mehreren davon, vorzugsweise Kondensation oder Destillation durchgeführt werden.

Im Falle einer mehrstufigen Stofftrennung kann die bei der ersten Stofftrennung gebildete Produktaldehyd-reiche Phase einer zweiten Stofftrennung, insbesondere einer nachfolgenden Aldehydabtrennung zugeführt werden, bei der das Produktaldehyd von den anderen in dieser Phase befindlichen Stoffen, häufig Alkane und Eduktolefine, abgetrennt wird. Die an nicht umgesetzten Olefin-reiche Phase kann zu dem Hydroformylierungsschritt oder in Falle einer mehrstufigen Ausgestaltung zu einer der Hydroformylierungsschritten zurückgeführt werden, um die darin enthaltenen Olefine auch noch zum Produktaldehyd zu hydroformylieren.

Bei der Stofftrennung kann neben den genannten Phasen auch ein Purgegasstrom entnommen werden, der eine zur an nicht umgesetzten Olefin-reiche Phase zumindest ähnliche oder identische Zusammensetzung aufweist. Der Purgegasstrom kann ebenfalls zur zweiten Stofftrennung bzw. Aldehydabtrennung geleitet werden, um die darin enthaltenen Produktaldehyde abzutrennen und um Verunreinigungen (z. B. Stickstoff im Synthesegas) oder inerte Substanzen (z. B. Alkane im Einsatzgemisch) aus dem System auszutragen. Die Verunreinigungen oder inerte Substanzen können üblicherweise bei der zweiten Stofftrennung als leichtflüchtige Stoffe, beispielsweise am Kopf einer Kolonne, abgenommen werden.

Ein weiterer Gegenstand der vorliegenden Erfindung ist auch eine Anlage mit der das vorliegende Verfahren durchgeführt werden kann und welches insbesondere einen Reaktor, in dem der erfindungsgemäße Hydroformylierungsschritt durchgeführt wird umfasst. Zusätzlich kann die Anlage eine Stofftrennungseinheit umfassen, mit der der gasförmige Austrag des Hydroformylierungsschritts in mindestens eine an nicht umgesetzten Olefin reiche Phase und mindestens eine Produktaldehyd-reiche Phase getrennt wird, wobei diese Stofftrennungseinheit nach dem erfindungsgemäßen Hydroformylierungsschritts angeordnet ist. Stromabwärts der ersten Stofftrennung kann eine zweite Stofftrennungseinheit, insbesondere eine Aldehydabtrennungseinheit, vorliegen, mit der der Produktaldehyd abgetrennt wird.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

### Beispiel:

### Versuch 1: Herstellung und Untersuchung eines nicht erfindungsgemäßen Katalysatorsystems mit ionischer Flüssigkeit

Als Support wurde ein Monolith aus Siliciumcarbid mit einer Länge von ca. 20 cm und einem Durchmesser von ca. 25 mm verwendet. Der Support war porös und wurde mit einem Washcoat (SiO₂) vorbehandelt. Der Support wies 31 Kanäle mit einem Durchmesser von ca. 3 mm auf. Der Support wurde in einen Reaktor eingesetzt und mit einer Katalysatorlösung, enthaltend Rh(acac)(CO)₂, Bisphephos (Ligand), Bis(2,2,6,6-tetramethyl-4-piperidyl)sebacat (Stabilisator), 1-Ethyl-3-methylimidazolium-bis(trifluoromethansulfonyl)imid ([EMIM][NTf₂] / ionische Flüssigkeit) und Dichlormethan als Lösemittel und hergestellt durch Mischen in einer inerten Umgebung (Glovebox), beaufschlagt. Dazu wurde die Katalysatorlösung nach dem Spülen des Reaktors mit Stickstoff mit leichtem Überdruck in den Reaktor eingeleitet. Nach Entfernen des Lösemittels aus dem Reaktor durch Ablassen und Verdampfen wurde das auf dem Support heterogenisierte Katalysatorsystem zur Hydroformylierung verwendet.

Als Einsatzgemisch wurde ein Kohlenwasserstoffstrom mit der folgenden Zusammensetzung verwendet:

| | Menge (Gew.-%) |
|---|---|
| 1-Buten / iso-Buten | 19,14 |
| cis-2-Buten | 19,10 |
| trans-2-Buten | 28,40 |
| n-Butan | 30,80 |
| iso-Butan | 0,02 |
| 2-Methyl-butan | 2,50 |

Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 °C und einem Druck von 10 bar durchgeführt. Der Gesamtumsatz an Butenen (also der Umsatz aller im Einsatzgemisch befindlichen Butene) und die n/iso-Selektivität (Verhältnis von linearen zu verzweigten Produkten) wurde gaschromatographisch über die Produktzusammensetzung ermittelt.

Nach einer Versuchsdauer von 500 Stunden betrug der Gesamtumsatz an Butenen 27% und die n/iso-Selektivität 98%.

### Versuch 2: Herstellung und Untersuchung eines erfindungsgemäßen Katalysatorsystems ohne ionische Flüssigkeit

Als Support wurde ein Monolith aus Siliciumcarbid mit einer Länge von ca. 20 cm und einem Durchmesser von ca. 25 mm verwendet. Der Support war porös und wurde mit einem Washcoat (SiO₂) vorbehandelt. Der Support wies 31 Kanäle mit einem Durchmesser von ca. 3 mm auf. Der Support wurde in einen Reaktor eingesetzt und mit einer Katalysatorlösung, enthaltend Rh(acac)(CO)₂, Biphephos (Ligand), Bis(2,2,6,6-tetramethyl-4-piperidyl)sebacat (Stabilisator) und Dichlormethan als Lösemittel und hergestellt durch Mischen in einer inerten Umgebung (Glovebox), beaufschlagt. Dazu wurde die Katalysatorlösung nach dem Spülen des Reaktors mit Stickstoff mit leichtem Überdruck in den Reaktor eingeleitet. Nach Entfernen des Lösemittels aus dem Reaktor durch Ablassen und Verdampfen wurde das auf dem Support heterogenisierte Katalysatorsystem zur Hydroformylierung verwendet.

Als Einsatzgemisch wurde ein Kohlenwasserstoffstrom mit nahezu identischer Zusammensetzung wie in Versuch 1 verwendet. Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 °C und einem Druck von 10 bar durchgeführt. Der Gesamtumsatz an Butenen (also der Umsatz aller im Einsatzgemisch befindlichen Butene) und die n/iso-Selektivität (Verhältnis von linearen zu verzweigten Produkten) wurde gaschromatographisch über die Produktzusammensetzung ermittelt.

Nach einer Versuchsdauer von 500 Stunden betrug der Gesamtumsatz an Butenen 56% und die n/iso-Selektivität 97%.

### Versuch 3: Herstellung und Untersuchung eines nicht erfindungsgemäßen SILP-Katalysatorsystems

Die Herstellung des Katalysatorsystems erfolgte analog zu der Herstellung der katalytisch aktiven Zusammensetzung Rh(ll) in der WO 2015/028284 A1.

Als Einsatzgemisch wurde ein Kohlenwasserstoffstrom mit der folgenden Zusammensetzung verwendet:

| | Menge (Gew.-%) |
|---|---|
| 1-Buten / iso-Buten | 27,40 |
| cis-2-Buten | 15,00 |
| trans-2-Buten | 25,00 |
| n-Butan | 29,50 |
| iso-Butan | 0,02 |
| 2-Methylbutan | 3,00 |

Das Einsatzgemisch wurde zusammen mit Synthesegas (molares Verhältnis Synthesegas : Einsatzgemisch = 3,5 : 1) zur Hydroformylierung mit einem Gasvolumenstrom von 390 ml/min in den Reaktor geleitet. Die Hydroformylierung wurde bei einer Temperatur von 120 °C und einem Druck von 10 bar durchgeführt. Der Gesamtumsatz an Butenen (also der Umsatz aller im Einsatzgemisch befindlichen Butene) und die n/iso-Selektivität (Verhältnis von linearen zu verzweigten Produkten) wurde gaschromatographisch über die Produktzusammensetzung ermittelt.

Nach einer Versuchsdauer von 500 Stunden betrug der Gesamtumsatz an Butenen 25% und die n/iso-Selektivität 92%.

Aus den Versuchsreihen ist somit zu erkennen, dass die erfindungsgemäßen heterogenisierten Katalysatorsystemen ohne ionische Flüssigkeit gegenüber den bekannten SILP-Systemen oder Systemen mit ionischer Flüssigkeit den Vorteil haben, dass damit höhere Umsätze und/oder eine höhere Linearität der Produkte (n/iso-Selektivität) erreicht werden können.

## Patentansprüche

1. Verfahren zur Hydroformylierung von C2- bis C5-Olefinen, wobei die Hydroformylierung einen oder mehrere Hydroformylierungsschritte umfasst, **dadurch gekennzeichnet, dass**
in mindestens einem Hydroformylierungsschritt ein Einsatzgemisch, welches die C2- bis C5-Olefine umfasst, mit Synthesegas in Gegenwart eines Katalysatorsystems, welches ein Metall aus der 8. oder 9. Gruppe des Periodensystems der Elemente, mindestens einen organischen Phosphor-enthaltenden Liganden und einen Stabilisator umfasst, in einer Reaktionszone einer Hydroformylierung unterworfen wird, wobei das Einsatzgemisch und das Synthesegas über einen Support aus einem porösen keramischen Material geleitet werden, auf dem das Katalysatorsystem heterogenisiert vorliegt,
das Katalysatorsystem keine ionische Flüssigkeit umfasst; und
der Support ein Monolith, das heißt ein Block aus einem keramischen Material ist, auf den ein Washcoat aus, bezogen auf das keramische Material des Supports, dem gleichen oder einem anderen keramischen Material aufgetragen wird,
wobei der Stabilisator eine organische Aminverbindung ist, die mindestens eine 2,2,6,6-Tetramethylpiperidineinheit nach Formel (I) enthält:

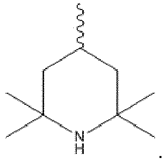

2. Verfahren nach Anspruch 1, wobei aus der Reaktionszone kontinuierlich ein gasförmiger Austrag entnommen wird, der mindestens einen Teil der entstandenen Produktaldehyde und mindestens einen Teil der nicht umgesetzten Olefine enthält und der gasförmige Austrag einem Stofftrennungsschritt unterworfen wird, bei der der gasförmige Austrag in mindestens eine an nicht umgesetzten Olefinen-reiche Phase und mindestens eine Produktaldehyd-reiche Phase getrennt wird.

3. Verfahren nach Anspruch 1 oder 2, wobei der organische Phosphor-enthaltende Ligand des Hydroformylierungskatalysatorsystems die allgemeine Formel (VI)
R' - A - Rʺ - A - R‴ (VI)
aufweist, wobei R', R" und R‴ jeweils organische Reste sind, mit der Maßgabe das R′ und R‴ nicht identisch sind, und beide A jeweils eine brückende -O-P(-O)₂-Gruppe sind, wobei zwei der drei Sauerstoffatome -O- jeweils an Rest R' und den Rest R‴ gebunden sind.

4. Verfahren nach einem der Ansprüche 1 bis 3, wobei das poröse keramische Material, aus dem der Support besteht, aus der Gruppe, bestehend aus einer Silikatkeramik, einer oxidischen Keramik, einer nitridischen Keramik, einer carbidischen Keramik, einer silicidischen Keramik und Mischungen davon, ausgewählt wird.

5. Verfahren nach Anspruch 4, wobei die Silikatkeramik ausgewählt wird aus Alumosilikat, Magnesiumsilikat und Mischungen davon, wie z. B. Bentonit; die oxidische Keramik ausgewählt wird aus γ-Atuminiumoxid, α-Aluminiumoxid, Titandioxid, Beryliumoxid, Zirkoniumoxid, Aluminiumtitanat, Bariumtitanat, Zinkoxid, Eisenoxiden (Ferrite) und Mischungen davon; die nitridische Keramik ausgewählt wird aus Siliciumnitrid, Bornitrid, Aluminiumnitrid und Mischungen davon; die carbidische Keramik ausgewählt wird aus Siliciumcarbid, Borcarbid, Wolframcarbid oder Mischungen davon; und die silicidische Keramik Molbydänsilicid ist.

6. Verfahren nach Anspruch 4, wobei das poröse keramische Material, aus dem der Support besteht, eine carbidische Keramik ist.

7. Verfahren nach Anspruch 5, wobei die carbidische Keramik ausgewählt wird aus Siliciumcarbid, Borcarbid, Wolframcarbid oder Mischungen davon.

8. Verfahren nach einem der Ansprüche 1 bis 7, wobei die Menge das auf dem Support befindlichen Waschcoats ≤ 20 Gew.-% beträgt, bezogen auf die Gesamtmenge des Supports.

9. Verfahren nach einem der Ansprüche 1 bis 8, wobei der Support aus dem keramischen Material einen oder mehrere Kanäle in Hauptdurchströmungsrichtung aufweist.

10. Verfahren nach einem der Ansprüche 1 bis 9, wobei der Hydroformylierungsschritt bei einer Temperatur im Bereich von 65 bis 200 °C durchgeführt wird.

11. Verfahren nach einem der Ansprüche 1 bis 10, wobei der Druck im Hydroformylierungsschritt nicht größer als 35 bar ist.

## Claims

1. Process for hydroformylating C2 to C5 olefins, wherein the hydroformylation comprises one or more hydroformylation steps, **characterized in that**
in at least one hydroformylation step a feed mixture comprising the C2 to C5 olefins is subjected to a hydroformylation with synthesis gas in the presence of a catalyst system comprising a metal from group 8 or 9 of the Periodic Table of the Elements, at least one organic phosphorus-containing ligand and a stabilizer, in a reaction zone, wherein the feed mixture and the synthesis gas are passed over a support composed of a porous ceramic material on which the catalyst system is in heterogenized form,
the catalyst system does not comprise any ionic liquid; and
the support is a monolith, i.e. a block of a ceramic material, to which a washcoat composed of the same or a different ceramic material with respect to the ceramic material of the support is applied,
wherein the stabilizer is an organic amine compound containing at least one 2,2,6,6-tetramethylpiperidine unit of formula (I):

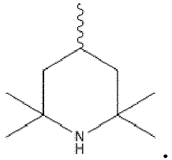

2. Process according to Claim 1, wherein a gaseous output comprising at least a portion of the product aldehydes formed and at least a portion of the unconverted olefins is withdrawn continuously from the reaction zone and the gaseous output is subjected to a physical separation step in which the gaseous output is separated into at least one phase rich in unconverted olefins and at least one phase rich in product aldehyde.

3. Process according to Claim 1 or 2, wherein the organic phosphorus-containing ligand in the hydroformylation catalyst system has the general formula (VI)
R′ - A - Rʺ - A - R‴ (VI)
where R′, Rʺ and R‴ are each organic radicals, with the proviso that R′ and R‴ are not identical, and each A is a bridging -O-P(-O)₂- group, where two of the three oxygen atoms -O- are respectively bonded to R' radical and R‴ radical.

4. Process according to any of Claims 1 to 3, wherein the porous ceramic material of which the support consists is selected from the group consisting of a silicate ceramic, an oxidic ceramic, a nitridic ceramic, a carbidic ceramic, a silicidic ceramic and mixtures thereof.

5. Process according to Claim 4, wherein the silicate ceramic is selected from aluminosilicate, magnesium silicate, and mixtures thereof, for example bentonite; the oxidic ceramic is selected from γ-alumina, α-alumina, titanium dioxide, beryllium oxide, zirconium oxide, aluminium titanate, barium titanate, zinc oxide, iron oxides (ferrites) and mixtures thereof; the nitridic ceramic is selected from silicon nitride, boron nitride, aluminium nitride and mixtures thereof; the carbidic ceramic is selected from silicon carbide, boron carbide, tungsten carbide or mixtures thereof; and the silicidic ceramic is molybdenum silicide.

6. Process according to Claim 4, wherein the porous ceramic material of which the support consists is a carbidic ceramic.

7. Process according to Claim 5, wherein the carbidic ceramic is selected from silicon carbide, boron carbide, tungsten carbide or mixtures thereof.

8. Process according to any of Claims 1 to 7, wherein the amount of the washcoat on the support is ≤ 20% by weight, based on the total amount of the support.

9. Process according to any of Claims 1 to 8, wherein the support composed of the ceramic material has one or more channels in main through-flow direction.

10. Process according to any of Claims 1 to 9, wherein the hydroformylation step is conducted at a temperature in the range from 65 to 200°C.

11. Process according to any of Claims 1 to 10, wherein the pressure in the hydroformylation step is not greater than 35 bar.

## Revendications

1. Procédé d'hydroformylation d'oléfines en C2 à C5, l'hydroformylation comprenant une ou plusieurs étapes d'hydroformylation, **caractérisé en ce que**
dans au moins une étape d'hydroformylation, on soumet dans une zone de réaction à une hydroformylation un mélange de charge qui comprend les oléfines en C2 à C5, avec un gaz de synthèse en présence d'un système catalyseur qui comprend un métal du 8e ou du 9e groupe du système périodique des éléments, au moins un ligand phosphoré organique et un stabilisant, le mélange de charge et le gaz de synthèse étant envoyés sur un support en un matériau céramique poreux, sur lequel le système catalyseur est présent sous forme hétérogénéisée,
le système catalyseur ne comprend aucun liquide ionique ; et
le support est un monolithe, c'est-à-dire un bloc d'un matériau céramique, sur lequel est appliquée une couche active, en un matériau céramique qui, par rapport au matériau céramique du support, est identique ou différent,
le stabilisant étant un composé aminé organique qui contient au moins un motif 2,2,6,6-tétraméthylpipéridine de formule (I) :

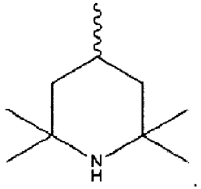

2. Procédé selon la revendication 1, dans lequel on soutire en continu de la zone de réaction un produit gazeux, qui contient au moins une partie des aldéhydes produits et au moins une partie des oléfines n'ayant pas réagi, et le produit gazeux est soumis à une étape de séparation, dans laquelle le produit gazeux est séparé en au moins une phase riche en oléfines n'ayant pas réagi et au moins une phase riche en aldéhydes produits.

3. Procédé selon la revendication 1 ou 2, dans lequel le ligand phosphoré organique du système catalyseur d'hydroformylation présente la formule générale (VI)
R′-A-Rʺ-A-R‴ (VI)
dans laquelle R′, Rʺ et R‴ représentent chacun des radicaux organiques, à la condition que R′ et R‴ ne soient pas identiques, et chacun des deux A représente un groupe pontant -O-P(-O)₂, deux des trois atomes d'oxygène -O- étant chacun lié au radical R′ et au radical R‴.

4. Procédé selon l'une des revendications 1 à 3, dans lequel le matériau céramique poreux dont est constitué le support est choisi dans le groupe consistant en une céramique de silicate, une céramique d'oxyde, une céramique de nitrure, une céramique de carbure, une céramique de siliciure et les mélanges de celles-ci.

5. Procédé selon la revendication 4, dans lequel la céramique de silicate est choisie parmi le silicate d'aluminium, le silicate de magnésium et les mélanges de ceux-ci, tels que par exemple la bentonite ; la céramique d'oxyde est choisie parmi l'oxyde d'aluminium y, l'oxyde d'aluminium a, le dioxyde de titane, l'oxyde de béryllium, l'oxyde de zirconium, le titanate d'aluminium, le titanate de baryum, l'oxyde de zinc, les oxydes de fer (ferrites) et les mélanges de ceux-ci ; la céramique de nitrure est choisie parmi le nitrure de silicium, le nitrure de bore, le nitrure d'aluminium et les mélanges de ceux-ci ; la céramique de carbure est choisie parmi le carbure de silicium, le carbure de bore, le carbure de tungstène ou les mélanges de ceux-ci ; et la céramique de siliciure est le siliciure de molybdène.

6. Procédé selon la revendication 4, dans lequel le matériau céramique poreux dont est constitué le support est une céramique de carbure.

7. Procédé selon la revendication 5, dans lequel la céramique de carbure est choisie parmi le carbure de silicium, le carbure de bore, le carbure de tungstène ou les mélanges de ceux-ci.

8. Procédé selon l'une des revendications 1 à 7, dans lequel la quantité de la couche active se trouvant sur le support est ≤ 20 % en poids par rapport à la quantité totale du support.

9. Procédé selon l'une des revendications 1 à 8, dans lequel le support en le matériau céramique comprend un ou plusieurs canaux dans la direction de l'écoulement principal.

10. Procédé selon l'une des revendications 1 à 9, dans lequel l'étape d'hydroformylation est mise en œuvre à une température dans la plage de 65 à 200 °C.

11. Procédé selon l'une des revendications 1 à 10, dans lequel la pression dans l'étape d'hydroformylation n'est pas supérieure à 35 bar.
